Europäisches Patentamt

European Patent Office

Office européen des brevets

(19)

(11) Numéro de publication: **0 041 030**
**B1**

(12) **FASCICULE DE BREVET EUROPEEN**

(45) Date de publication du fascicule du brevet:
14.03.84

(51) Int. Cl.³: **A 61 K 7/48**

(21) Numéro de dépôt: **81400852.0**

(22) Date de dépôt: **27.05.81**

(54) **Compositions à usage topique contenant des alcaloides de l'ergot de seigle et de vinca rosea destinés au traitement des hyperséborrhées, ainsi que leur préparation.**

(30) Priorité: **28.05.80 FR 8012166**

(43) Date de publication de la demande:
**02.12.81 Bulletin 81/48**

(45) Mention de la délivrance du brevet:
**14.03.84 Bulletin 84/11**

(84) Etats contractants désignés:
**AT BE CH DE FR GB IT LI LU NL SE**

(56) Documents cités:
**FR - A - 2 276 832**
**FR - A - 2 313 059**
**US - A - 3 749 784**

**CHEMICAL ABSTRACTS, vol. 73, no. 7, 17 août 1970, page 206, abrégé no. 33837p, COLUMBUS OHIO (US)**
**CHEMICAL ABSTRACTS, vol. 85, no. 5, 2 août 1976, page 32, abrégé no. 28628c, COLUMBUS OHIO (US)**
**THE MERCK INDEX, édition 8, 1968, MERCK & CO., RAHWAY N.J. (US), pages 1107-1108**

(73) Titulaire: **PIERRE FABRE S.A., 125, rue de la Faisanderie, F-75116 Paris (FR)**

(72) Inventeur: **Navarro, Roger, Chemin des Farguettes Lambert, F-81100 Castres (FR)**
Inventeur: **Franceschini, P., 17 avenue Jean Moulin, F-81106 Castres Cedex (FR)**

(74) Mandataire: **Corre, Jacques Denis Paul et al, Cabinet Regimbeau 26, Avenue Kléber, F-75116 Paris (FR)**

BUNDESDRUCKEREI BERLIN

# Compositions a usage topique contenant des alcaloides de l'ergot de seigle et de vinca rosea destines au traitement des hyperseborrhees, ainsi que leur preparation

L'invention, réalisée au Centre de Recherche Pierre Fabre, a pour objet des médicaments utiles notamment pour le traitement des états hyperséborrhéiques tels que les acnés et les alopécies séborrhéiques.

Ces médicaments sont constitués par des compositions pharmaceutiques, administrables par voie externe, contenant des principes actifs en combinaison avec un support pour application topique.

Les principes actifs utilisés dans le cadre de l'invention sont des alcaloïdes de l'ergot de seigle, en particulier les dérivés hydrogénés tels que la dihydroergotamine et la dihydroergocristine, les dérivés d'hémisynthèse tel que la bromocriptine, et des alcaloïdes de Vinca rosea, en particulier la raubasine et la tétrahydroalstonine. L'invention vise également les sels pharmaceutiquement acceptables de ces alcaloïdes.

Ces substances sont bien connues pour leurs propriétés vasculotropes et sont largement utilisées dans le domaine thérapeutique en particulier pour la prévention et le traitement des troubles circulatoires et vasculaires tels qu'insuffisances veineuses, ischémies, migraines, etc. Les médicaments contenant ces substances ont été jusqu'à présent administrés uniquement par voie interne. Les compositions pharmaceutiques selon la présente invention sont du type général renfermant au moins un alcaloïde de Vinca rosea avec un alcaloïde de l'ergot de seigle. Ces alcaloïdes sont connus dans la technique antérieure et notamment pour l'usage particulier envisagé dans le cadre de la présente invention. L'état de la technique antérieure peut par exemple être illustré par FR-A-2 313 059, US-A-3 749 784, FR-A-2 276 832 et The Merck Index, 8ème édition, 1968, Merck & Co, Rahway, N.J., USA, pages 1107 et 1108.

Les compositions pharmaceutiques selon la présente invention sont caractérisées en ce qu'elles contiennent en combinaison, au moins un principe actif représentant jusqu'à 4% en poids de la composition et un support, le principe actif comprenant 0,05 à 1% en poids de la composition de tétrahydroalstonine, ou de l'un de ses sels pharmaceutiquement acceptables et, éventuellement, aussi au moins un autre alcaloïde de Vinca rosea ou un alcaloïde de l'ergot de seigle connu en soi pour ledit usage et le support assurant la pénétration et la rémanence du principe actif au niveau des glandes sébacées.

Le support doit assurer le passage à travers les matières obstruant les orifices pilo-sébacés et la rémanence des principes actifs au niveau des sites, notamment des glandes sébacées. Il est remarquable de constater que ces nouvelles compositions permettent de traiter des affections qui relèvent d'un domaine thérapeutique totalement différent de celui dans lequel les principes actifs étaient utilisés jusqu'à présent. Par ailleurs, il faut noter que les nouvelles compositions administrables par voie topique ont pour unique propriété de normaliser l'état des peaux hyperséborrhéiques. Elles sont donc utiles spécifiquement pour le traitement des affections cutanées telles que les acnés et les alopécies séborrhéiques.

Le support est remarquable en ce qu'il permet de dissoudre les principes actifs et de les amener au niveau de la glande sébacée à travers ses propres sécrétions, particulièrement abondantes dans les cas d'hyperséborrhées. Ce support est constitué essentiellement par la combinaison de 20 à 70% en volume d'alcool éthylique, le solde étant formé soit d'eau, soit de glycérol, soit d'un polyol tel que l'éthylène glycol, le propylène glycol ou leurs homologues oxyéthylinés pris seuls ou en mélange. Peuvent être également associés en petites quantités (inférieures à 10% de la composition) des cétones (telles que la méthyl isobutylcétone) des esters (tels que l'acétate d'éthyle) ou des éthers (tels qu le diméthoxyméthane) permettent de concourir à l'effet recherché sans causer d'effets désagréables au niveau de la peau.

Les formes galéniques des compositions selon l'invention peuvent être des solutions, des lotions ou des gels en flacon, en aérosol ou en ampoules. Les compositions contiennent de préférence de 0,01% à 4% en poids de principes actifs, le principe actif comprenant 0,05 à 1% en poids de la composition de tétrahydroalstonine, ou l'un de ses sels pharmaceutiquement acceptables.

Conformément à la présente invention, le principe actif peut en outre contenir au moins un composé choisi parmi la dihydroergotamine, la dihydroergocristine, la raubasine, la bromocriptine et leurs sels pharmaceutiquement acceptables.

Les compositions peuvent contenir plusieurs alcaloïdes et leurs sels pharmaceutiquement acceptables en association, ainsi que des principes actifs à activité antiseptique, anti-inflammatoire ou antifongique.

A titre d'exemple, la préparation d'une composition selon l'invention est décrite:

Dans un récipient en acier inoxydable fermé et muni d'un agitateur, on introduit 40 litres d'alcool éthylique à 96°, puis 0,4 kg de dihydroergotamine méthane sulfonate. On fait le vide auparavant puis on introduit de l'azote dont un barbotage sera assuré pendant toute la durée des opérations. On rajoute sous agitation 6 kg de glycérine puis une partie de l'eau. Le parfum (50 g) en mélange avec son émulsionnant, qui pourra être de l'huile de ricin éthoxylée à raison de 100 g, est ensuite introduit, puis le colorant, par exemple du bleu patenté (4 mg de poudre au préalable dissous dans un peu d'eau). Le complément à 100 litres est réalisé avec de l'eau distillée et l'agitation poursuivie jusqu'à homogénéisation complète.

Cette composition contenant la dihydroergotamine méthane sulfonate a fait l'objet d'expérimentations pharmacologiques et cliniques.

Des tests sur des cobayes, avec aplication de la composition, sur le flanc préalablement tondu, trois fois par jour pendant 6 semaines n'ont révélé aucune intolérance. Toutes les observations effectuées montrent qu'il n'y a ni irritation primaire, ni phénomène de sensibilisation.

Des essais ont été pritiqués sur des patients présentant des acnés accompagnées d'hyperséborrhées ou d'alopécies séborrhéiques. Les préparations pharmaceutiques contenant la dihydroergotamine ont été appliquées une à deux fois par jour, sur le dos, le visage et le cuir chevelu. La durée du traitement a varié de quatre à douze semaines, en fonction de la gravité de l'état hyperséborrhéique. Dans de très nombreux cas, une amélioration notable des signes cliniques a été constatée.

A titre d'exemples non limitatifs de compositions pharmaceutiques selon l'invention, quelques autres exemples sont cités:

### Exemple 1

| | | |
|---|---|---|
| Tétrahydroalstonine base | 1 g | |
| Excipient hydroalcoolique (50/50) | qsp 100 ml | |

### Exemple 2

| | | |
|---|---|---|
| Tétrahydroalstonine base | 0,5 g | |
| Dihydroergotamine méthane sulfonate | 0,5 g | |
| Excipient polyoxyéthylène glycol 400 | 6 g | |
| Alcool éthylique | qsp 100 ml | |

### Exemple 3

| | | |
|---|---|---|
| Tétrahydroalstonine base | 0,05 g | |
| Bromocriptine méthane sulfonate | 0,10 g | |
| Dihydroergotamine méthane sulfonate | 0,30 g | |
| Excipient hydroalcoolique (50/50) | qsp 100 ml | |

Conditionnement Aérosol propulsé par un mélange de carbures chloro fluorés.

La présente invention concerne également un procédé de préparation des compositions pharmaceutiques selon l'invention consistant à mélanger au moins un principe actif précédemment cité à un support assurant la pénétration et la rémanence du principe actif au niveau des glandes sébacées. Comme indiqué précédemment, un tel support est de préférence constitué essentiellement par la combinaison de 20 à 70% en volume d'alcool éthylique et d'un autre constituant choisi parmi l'eau, le glycérol, un polyol tel que l'éthylène glycol, le propylène glycol ou leurs homologues oxyéthylinés.

La présente invention ne saurait être limitée aux exemples décrits ci-dessus. En outre, les principes actifs alcaloïdes des compositions selon l'invention ainsi que leurs dérivés ou sels pharmaceutiquement acceptables peuvent parfaitement être obtenus à partir d'autres espèces végétales telles que Vinca Lancéa ou Rauwolfia ou encore par voie entièrement synthétique.

## Revendications pour les Etats contractants: BE, CH, DE, FR, GB, IT, LI, LU, NL, SE

1. Compositions pharmaceutiques administrables par voie topique pour usage notamment dans le traitement des acnés et des alopécies séborrhéiques et du type renfermant au moins un alcaloïde de Vinca rosea, caractérisées en ce qu'elles contiennent en combinaison, au moins un principe actif représentant jusqu'à 4% en poids de la composition et un support, le principe actif comprenant 0,05 à 1% en poids de la composition de tétrahydroalstonine, ou de l'un de ses sels pharmaceutiquement acceptables et, éventuellement, aussi au moins un autre alcaloïde de Vinca rosea ou un alcaloïde de l'ergot de seigle connu en soi pour ledit usage et le support assurant la pénétration et la rémanence du principe actif au niveau des glandes sébacées.

2. Compositions pharmaceutiques selon la revendication 1, caractérisées en ce que ledit alcaloïde de Vinca rosea ou de l'ergot de seigle est choisi parmi la dihydroergotamine, la dihydroergocristine, la raubasine, la bromocriptine et leurs sels pharmaceutiquement acceptables.

3. Compositions pharmaceutiques selon l'une des revendications 1 et 2, caractérisées en ce qu'elles contiennent en plus, à titre de principes actifs associés, un ou plusieurs composés pris dans les classes des antiseptiques, des anti-inflammatoires ou des antifongiques.

4. Compositions pharmaceutiques selon l'une des revendications 1 à 3, caractérisées en ce que l'on mélange ledit principe actif avec un support assurant la pénétration et la rémanence du principe actif au niveau des glandes sébacées.

5. Compositions pharmaceutiques selon la revendication 4, caractérisées en ce que ledit support est constitué par un mélange de 20 à 70% en volume d'alcool éthylique et d'au moins un autre constituant choisi parmi l'eau, le glycérol, un polyol tel que l'éthylène glycol, le propylène glycol et leurs homologues oxyéthylinés.

## Revendications pour l'Etat contractant: AT

1. Procédé de préparation de compositions pharmaceutiques administrables par voie topique pour usage notamment dans le traitement des acnés et des alopécies séborrhéiques et du type renfermant au moins un alcaloïde de Vinca rosea, caractérisé en ce que l'on mélange au moins un principe actif représentant jusqu'à 4% en poids de la composition avec un support assurant la pénétration et la rémanence du principe actif au niveau des glandes sébacées, ledit principe actif comprenant 0,05 à 1% en poids de la composition de tétrahydroalstonine ou de l'un de ses sels pharmaceutiquement acceptables et, éventuellement, aussi au moins un autre alcaloïde de Vinca rosea ou un alcaloïde de l'ergot de seigle connu en soi pour ledit usage.

2. Procédé selon la revendication 1, caractérisé en ce que ledit principe actif contient en outre au moins un composé choisi parmi la dihydroergotamine, la dihydroergocristine, la raubasine, la bromocriptine et leurs sels pharmaceutiquement acceptables.

3. Procédé selon l'une des revendications 1 et 2, caractérisé en ce que l'on mélange ledit principe actif et le support avec un ou plusieurs principes actifs associés pris dans la classe des antiseptiques, des anti-inflammatoires ou des antifongiques.

4. Procédé selon l'une des revendications 1 à 3, caractérisé en ce que ledit support est constitué par un mélange de 20 à 70% en volume d'alcool éthylique et d'au moins un autre constituant choisi parmi l'eau, le glycérol, un polyol tel que l'éthylène glycol, le propylène glycol et leurs homologues oxyéthylinés.

## Patentansprüche für die Vertragsstaaten: BE, CH, DE, FR, GB, IT, LI, LU, NL, SE

1. Auf topischem Wege verabreichbare pharmazeutische Zusammensetzungen zur Verwendung insbesondere bei der Behandlung von Akne und Haarausfall durch Seborrhoe und von der Art, die mindestens ein Alkaloid von Vinca rosea aufweist, dadurch gekennzeichnet, daß sie in Kombination mindestens einen Wirkstoff, der bis zu 4 Gew.-% der Zusammensetzung darstellt, und einen Träger enthalten, wobei der Wirkstoff 0,05 bis 1 Gew.-%, bezogen auf die Zusammensetzung, Tetrahydroalstonin oder eines der pharmazeutisch annehmbaren Salze hiervon und gegebenfalls auch mindestens ein anderes Alkaloid von Vinca rosea oder ein an sich für die genannte Verwendung bekanntes Mutterkornalkaloid umfaßt und der Träger das Eindringen und das Verbleiben des Wirkstoffes in die bzw. an den Talgdrüsen gewährleistet.

2. Pharmazeutische Zusammensetzungen gemäß Anspruch 1, dadurch gekennzeichnet, daß das genannte Alkaloid von Vinca rosea oder des Mutterkorns unter Dihydroergotamin, Dihydroergocristin, Raubasin, Bromcryptin und deren pharmazeutisch annehmbaren Salzen gewählt ist.

3. Pharmazeutische Zusammensetzungen gemäß einem der Ansprüche 1 und 2, dadurch gekennzeichnet, daß sie außerdem als weitere Wirkstoffe eine oder mehrere Verbindungen aus den Klassen der antiseptischen, der entzündungshemmenden oder der Antipilz-Mittel enthalten.

4. Pharmazeutische Zusammensetzungen gemäß einem der Ansprüche 1 bis 3, dadurch gekennzeichnet, daß man den genannten Wirkstoff mit einem Träger mischt, der das Eindringen und das Verbleiben des Wirkstoffes in die bzw. an den Talgdrüsen gewährleistet.

5. Pharmazeutische Zusammensetzungen gemäß Anspruch 4, dadurch gekennzeichnet, daß der genannte Träger durch eine Mischung von 20 bis 70 Vol.-% Äthylalkohol und mindestens einem anderen Bestandteil ausgewählt unter Wasser, Glycerin, einem Polyol, wie Äthylenglykol, Propylenglykol und deren oxyäthylierten Homologen gebildet ist.

## Patentansprüche für den Vertragsstaat: AT

1. Verfahren zur Herstellung von auf topischem Wege verabreichbaren pharmazeutischen Zusammensetzungen zur Verwendung insbesondere bei der Behandlung von Akne und Haarausfall durch Seborrhoe und von der Art, die mindestens ein Alkaloid von Vinca rosea aufweist, dadurch gekennzeichnet, daß man mindestens einen Wirkstoff, der bis zu 4% Masse der Zusammensetzung darstellt, mit einem Träger, der das Eindringen und das Verbleiben des Wirkstoffes in die bzw. an den Talgdrüsen gewährleistet, mischt, wobei der Wirkstoff 0,05 bis 1% Masse, bezogen auf die Zusammensetzung, Tetrahydroalstonin oder eines der pharmazeutisch annehmbaren Salze hiervon und gegebenenfalls auch mindestens ein anderes Alkaloid von Vinca rosea oder ein für diesen Zweck an sich bekanntes Mutterkornalkaloid umfaßt.

2. Verfahren gemäß Anspruch 1, dadurch gekennzeichnet, daß der genannte Wirkstoff außerdem mindestens eine Verbindung ausgewählt unter Dihydroergotamin, Dihydroergocristin, Raubasin, Bromcryptin und deren pharmazeutisch annehmbaren Salzen enthält.

3. Verfahren gemäß einem der Ansprüche 1 und 2, dadurch gekennzeichnet, daß man den genannten Wirkstoff und den Träger mit einem oder mehreren weiteren Wirkstoffen aus der Klasse der antiseptischen, der entzündungshemmenden oder der Antipilz-Mittel mischt.

4. Verfahren gemäß einem der Ansprüche 1 bis 3, dadurch gekennzeichnet, daß der genannte Träger durch eine Mischung von 20 bis 70 Vol.-% Äthylalkohol und mindestens einem anderen Bestandteil ausgewählt unter Wasser, Glycerin, einem Polyol, wie Äthylenglykol, Propylenglykol und deren oxyäthylierten Homologen gebildet wird.

## Claims für the Contracting States: BE, CH, DE, FR, GB, IT, LI, LU, NL, SE

1. Pharmaceutical compositions administrable topically for use particularly in the treatment of acnes or seborrhoeric alopecias and of the type including at least one Vinca Rosea alkaloid, characterised in that they contain in combination, at least one active constituent representing up to 4% by weight of composition and a carrier, the active constituent comprising 0.05 to 1% by weight of the composition of tetrahydroalstonine, or one of its pharmaceutically acceptable salts and posssibly also at least one other Vinca Rosea alkaloid or an Ergot alkaloid known in itself for the said use and the carrier assuring penetration and retention of the active constituent in the region of the sebaceous glands.

2. Pharmaceutical compositions according to claim 1, characterised in that the said Vinca Rosea or Ergot alkaloid is chosen from dihydroergotamine, dihydroergocristine, raubasine, bromocriptine and their pharmaceutically acceptable salts.

3. Pharmaceutical compositions according to either of claims 1 and 2, characterised in that they contain in addition, by way of associated active constituents, one or more compounds taken from the classes of antiseptics, antiinflammatory agents, and anti-fungus agents.

4. Pharmaceutical compositions according to one of claims 1 to 3, characterised in that the said active constituent is mixed with a carrier assuring penetration and retention of the active constituent at the level of the sebaceous glands.

5. Pharmaceutical compositions according to claim 4, characterised in that the said carrier is constituted by a mixture of 20 to 70% by volume of ethylene alcohol and at least one other constituent chosen from water, glycerol, a polyol such as ethylene glycol, propylene glycol and their oxyethylene homologues.

**Claims for the Contracting State: AT**

1. Process for the preparation of pharmaceutical propositions administrable topically for use particularly in the treatment of acnes and seborrhoeric alopecias and of the type including at least one Vinca Rosea alkaloid, characterised in that at least one principal constituent representing up to 4% by weight of the composition is mixed with a carrier assuring penetration and retention of the active constituent in the region of the sebaceous glands, the said principal constituent having from 0.05 to 1% by weight of the composition of tetrahydroalstonine or one of its pharmaceutically acceptable salts and, possibly, also at least one other Vinca Rosea alkaloid known in itself for the said use.

2. Process according to claim 1, characterised in that the said principal constituent contains in addition at least one compound chosen from a dihydroergotamine, dihydroergocristine, raubasine, bromocriptine and their pharmaceutically acceptable salts.

3. Process according to either of claims 1 and 2, characterised in that the principal constituent and the carrier are mixed with one or more associated principal constituents taken from the class of antiseptics, antiinflammation agents or anti-fungus agents.

4. Process according to any one of claims 1 to 3, characterised in that the said carrier is constituted by a mixture of 20 to 70% by volume of ethyl alcohol and at least one other constituent chosen from water, glycerol, a polyol such as ethylene glycol, propylene glycol and their oxyethylene homologues.